## Europäisches Patentamt

## European Patent Office

## Office européen des brevets

(11) Veröffentlichungsnummer: **0 151 782**
**B1**

(12) # EUROPÄISCHE PATENTSCHRIFT

(45) Veröffentlichungstag der Patentschrift:
**27.04.88**

(51) Int. Cl.⁴: **B 01 J 2/10,** F 26 B 5/04,
A 61 K 9/16, B 01 J 2/16

(21) Anmeldenummer: **84115987.4**

(22) Anmeldetag: **20.12.84**

(54) **Verfahren und Vorrichtung zur Wärmebehandlung von Pulver oder Granulat.**

(30) Priorität: **21.12.83 AT 44651/83**

(43) Veröffentlichungstag der Anmeldung:
**21.08.85 Patentblatt 85/34**

(45) Bekanntmachung des Hinweises auf die Patenterteilung:
**27.04.88 Patentblatt 88/17**

(84) Benannte Vertragsstaaten:
**AT BE CH DE FR GB IT LI NL SE**

(56) Entgegenhaltungen:
**EP-A-0 080 199**
**DE-A-2 218 729**
**DE-A-2 942 325**
**DE-A-3 149 421**

(73) Patentinhaber: **Gergely, Gerhard, Dr., Gartengasse 8, A-1050 Wien (AT)**

(72) Erfinder: **Gergely, Gerhard, Dr., Gartengasse 8, A-1050 Wien (AT)**

(74) Vertreter: **Büchel, Kurt F., Dr., Patentanwalt Dr. Kurt F. Büchel Bergstrasse 297, FL- 9495 Triesen (LI)**

## Beschreibung

Die Erfindung betrifft ein Verfahren und eine Vorrichtung nach dem Oberbegriff des Anspruches 1.

Solche Verfahren sind in der Vergangenheit insbesondere zum Trocknen bekanntgeworden, z. B. nach der EP-A-80 199, der US-A-4 535 550 oder der DE-A-29 42 325. Sie haben den Nachteil, dass es - z. B. für eine vorangegangene Beschichtung, Granulierung o.dgl. - gar nicht so einfach ist, das Beschichtungs- bzw. Granulierungsmittel gleichmässig in der Masse zu verteilen.

Nun ist auch bereits vorgeschlagen worden, dieses Mittel in flüssiger, gelöster oder in Dampfform in die Trommel einzusaugen, um die gewünschte Behandlung zu erzielen, bevor wieder getrocknet wird, z. B. nach der DE-C-1 060 093, der DE-A-22 18 729 oder der AT-B-372 299, oder in die Trommel einzudüsen, z. B. nach der bereits erwähnten EP-A-80 199. Dabei konzentriert sich das Behandlungsmittel aber an der Stelle des ersten Auftreffens auf die Partikel und bildet z. B. dort zunächst grössere Bröckchen, wobei es dann schwierig oder in manchen Fällen unmöglich ist, das Behandlungsmittel anschliessend gleichmässig in der Masse zu verteilen.

Erfindung hat sich daher zur Aufgabe gestellt, nach Wegen zu suchen, die zwei Erfordernissen gerecht werden:

1. der leichten und ubiquitären Verteilung eines Behandlungs-, z. B. eines Lösungsmittels in dem Behandlungsgut;

2. der verstärkten Zufuhr von Wärmeenergie zu dem Behandlungsgut, gegebenenfalls auch im Vakuum.

Diese Aufgabe wird erfindungsgemäss durch das Kennzeichen des Anspruches 1 gelöst. Wird nämlich das Behandlungsmittel ohne Trägergas und nur an einer Stelle eingeschleust, dann reagiert es bevorzugt mit den Teilchen des Behandlungsgutes, auf die es zuerst trifft. Eine gleichmässige Behandlung ist dabei sehr erschwert. Selbst das Anlegen von Vakuum, das den Behandlungsvorgang jedenfalls vergleichmässigt, reicht in vielen Fällen nicht aus, um die Behandlung kontrollierbar und reproduzierbar zu machen.

Der Gasstrom wird zwar in den meisten Fällen Luft sein, doch kann für bestimmte Anwendungsfälle, z. B. wenn Sauerstoff ausgeschlossen werden soll, selbstverständlich in analoger Weise mit Stickstoff, Argon oder Kohlendioxid gearbeitet werden.

Besonders vorteilhafte Ausführungsformen des erfindungsgemässen Verfahrens sind in den Unteransprüchen 2 bis 5 beschrieben.

Im Prinzip beruht die Erfindung darauf, dass z. B. erhitzte Luft mittels eines sehr geringen Vakuums durch das Behandlungsgut hindurchgesaugt bzw. -gepresst wird, wobei am Eingang des Wärmeaustauschers, der die Luft erhitzt, ein Gefäss angebracht ist, das unter gleichen Druckverhältnissen der Einströmbedingungen Lösungsmittel in den Luftstrom eintropft oder zufliessen lässt. Hiebei muss die Temperatur des Wärmeaustauschers etwas höher sein als der Siedepunkt des entsprechenden Lösungsmittels. Das Lösungsmittel gelangt in den Wärmeaustauscher, verdampft dort und gelangt zusammen mit der heissen Luft in den Strömungseingang des Vakuum-Mischkessels. Es ist zwar durchaus möglich, den Strömungseingang an einen solchen Punkt zu legen, an dem eine möglichst grosse Säule des Pulvergemisches durchströmt wird. Das gelingt bei einem Vakuum-Mischkessel, der so eingestellt werden kann, dass bei einer Neigung von etwa 30 Grad der Eintritt des Luft-Dampf-Gemisches am untersten Punkt der im Kessel befindlichen Pulvermenge liegt, oder mit Hilfe eines an sich bekannten Lochrührers. In beiden Fällen kann eine besonders gleichmässige Verteilung des Lösungsmitteldampfes im gesamten Pulvergemisch erfolgen.

Bei gleichzeitigem Rühren des Pulvergemisches wird nun die heisse Luft zusammen mit dem Lösungsmitteldampf an der Pulvermasse abgekühlt und es kommt zum Kondensieren des Lösungsmitteldampfes an und in der Pulveroberfläche. Die hiebei abgegebene Verdampfungswärme wird dem Produkt zugeführt und erhöht die Produkttemperatur. Auf diese Weise ist es insbesondere möglich, sehr leicht und mit geringem Lösungsmittelaufwand Teile des Pulvergemisches - insbesondere die Oberfläche der Pulverteilchen - zu Granulierungszwecken anzulösen, oder auch in dem Pulvergemisch vorhandene Bindemittel aufzulösen, so dass eine Zuführung von Lösungen unnötig wird.

Die durch die Kondensationsenergie zugebrachte Wärmemenge ist sodann meist aureichend, um bei Anlegung des Vakuums das Lösungsmittel wiederum zu verdampfen.

Man kann auf diese Weise, durch Anlegen eines bestimmten Vakuums, den Vorgang kontinuierlich gestalten, so dass zunächst eine gewisse Lösungsmittelmenge in der Pulvermenge kondensiert und ab Erreichung einer bestimmten Temperatur das frisch einströmende Lösungsmittel wiederum verdampft und im nachgeschalteten Kondensator kondensiert wird. Man kann auf diese Weise auch kontinuierliche Granulationsvorgänge erreichen.

Dieses Verfahren ist auch geeignet, Kristallwasser aus Substanzen zu entfernen, wenn sie beispielsweise durch Alkohol aus dem Molekül "herausgelöst" werden können. Beispielsweise gelingt die partielle Entwässerung von Lactose oder die Kristallwasserbefreiung von Ampicillin in sehr eleganter Weise mittels Durchströmen von heissem, gesättigtem Isopropanoldampf unter Begleitung heisser Luft.

Ein insbesondere bei Vakuumbehältern auftretender Mangel ist die schlechte Übertragung von Wärme von der geheizten Behälterwand oder der nur geringen Wärmeinhalt aufweisenden Trocknungsluft auf das zu behandelnde Gut.

Abgesehen von der genannten enormen Energie- und Zeiteinsparung ist ein durch die vorliegende Erfindung gebotener wesentlicher Vorteil die Reinhaltung der Wand des Rühr- und Behandlungsbehälters. Dabei lässt sich sogar ein Reinigen der Wand während der Mischvorgänge durch einströmende oder implodierende Dampf-Luft-Mischungen erzielen.

In besonders vorteilhafter Weise lässt sich das erfindungsgemässe Verfahren für die Dampfgranulation verwenden, insbesondere wenn es im Vakuum betrieben wird und der Mischkessel in seiner Vertikalebene um seine horizontale Längsachse zur Durchführung einer Pendelbewegung befähigt ist, wobei die Rührorgane das im Behälter enthaltene Gut jeweils entgegen der Schwerkraft fördern sollen.

Die Erfindung wird an Hand der beigefügten Zeichnung sowie anschliessender Beispiele näher erläutert, wobei schon jetzt darauf hinzuweisen ist, dass die gegebenen Daten in keiner Weise einschränkend zu verstehen sind. Es zeigen:

Fig. 1 eine Anlage zur Durchführung einer ersten Ausführungsform des erfindungsgemässen Verfahrens in schematischer Anordnung;

Fig. 2 einen Vakuumrühr- und -behandlungsbehälter zur Durchführung einer zweiten Ausführungsform des erfindungsgemässen Verfahrens, schematisch im Schnitt.

Ein Vakuummischer 1 ist einerseits über einen Lösungsmittelkondensator 2 an die Vakuumpumpe 3 angeschlossen. Der Kondensator 2 besitzt Kühlwasserzu- und -abläufe 7 und einen Kondensatablass 8. Andererseits steht der schräggestellte Vakuumbehälter 1 über ein Gas- bzw. Dampfeinlassventil 4 mit dem Wärmeaustauscher 5 in Verbindung, in dem von einem Kompressor über das Zuführventil 18 gelieferte Luft erwärmt und aus dem Lösungsmittelzuführgerät 6 mit einem Behandlungsmittel in Dampfform beaufschlagt wird. In den Beispielen wird das Arbeiten mit dieser Anlage beschrieben.

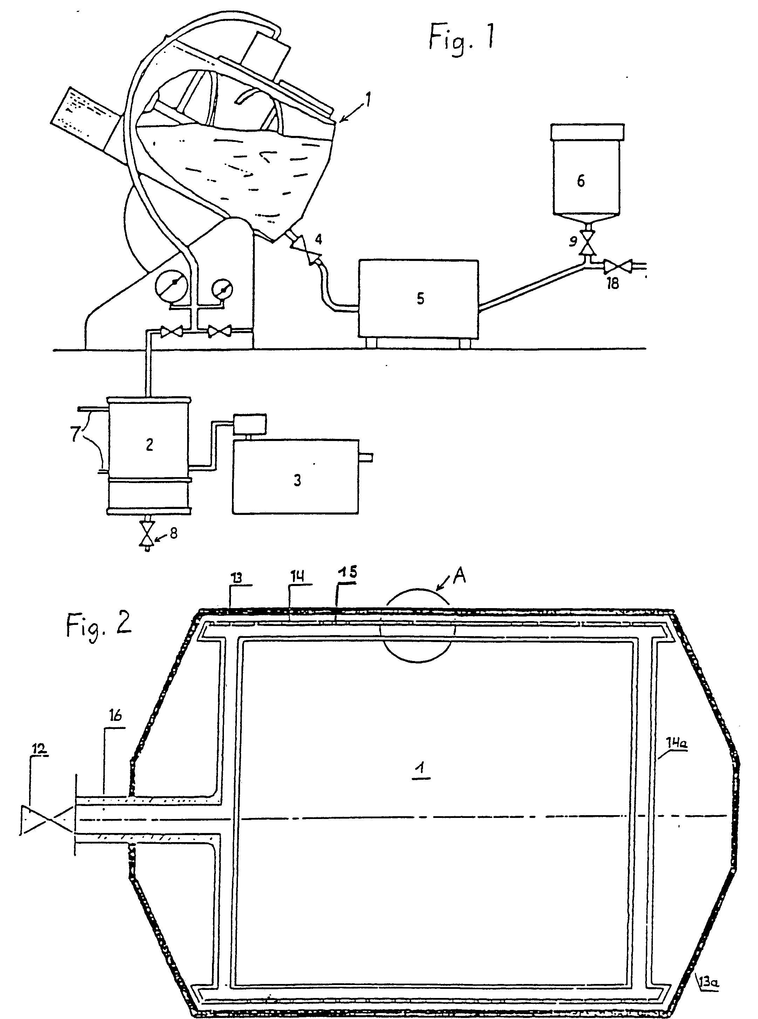

Nach einer zweiten Ausführungsform der Erfindung ist der im Vakuumbehälter 1 befindliche Rührer 14, 14a (Fig. 2) hohl ausgebildet. Der Behälter 1 weist eine gegebenenfalls heizbare Wand 13 auf. In den Behälter mündet abgedichtet eine Hohlwelle 16, an welche der aus hohlen Balkenelementen zusammengesetzte Rührer 14 angeschlossen ist. Der Hohlwelle 16 ist ein Ventil 12 vorgeschaltet. In der Behälterwand 13 gegenüberliegenden Teilen des hohl ausgebildeten Rührelements sind gegen die Behälterwand weisende Austrittsöffnungen 15 in Form von Löchern oder Schlitzen ausgebildet, durch welche das durch die Hohlwelle 16 eingespeiste Behandlungsmedium in den Behälter eingebracht wird.

Es ist offensichtlich, dass auch in dem dem kegelstumpfförmigen Boden 13a des Behälters gegenüberliegenden Teil 14a des Rührers Austrittsöffnungen vorgesehen sein können und dieser Rührerteil dem Verlauf des Bodens 13a angepasst sein kann, so dass auch hier dieselbe Wirkung wie bei den zylindrischen Seitenwänden erzielt wird. Desgleichen ist es, wie gesagt, auch möglich, auf der Hohlwelle und auf den gegen das Behälterinnere weisenden Seiten der Rührelemente Austrittsöffnungen vorzusehen.

Es ist auch denkbar, den Hohlrührer in einem zylindrischen Behälter (1) zu verwenden, der als Wirbelschichttrockner ausgebildet ist, wobei die Trockenluft in den Trockenbehälter statt durch das üblicherweise vorgesehene Netz durch die Austrittsöffnungen der Rührorgane eintritt.

**Beispiel 1** (Granulation von Staubzucker):

Bei einem Kesselinhalt von 200 kg Staubzucker sind 4 bis 5 Liter Wasser im Lösungsmittelzuführgerät (6) nötig. Wenn der Staubzucker eine Produkttemperatur von 60 Grad C erreicht hat und der Wärmeaustauscher auf 120 Grad C ist, wird unter einem Überdruck von 0,2 bis 0,5 bar am Kompressor Luft eingeleitet und die Vakuumpumpe bei Öffnung des Zuströmventiles (4) so eingestellt, dass das am Kessel anliegende Vakuum etwa 800 mbar beträgt.

Sodann wird der Hahn (9) geöffnet und die Wassermenge zur Granulierung in einem Zeitraum von 10 bis 20 Minuten in das System einfliessen gelassen. Der bei (5) entwickelte Wasserdampf durchströmt über das Ventil (4) den Staubzucker im Vakuum-Mischkessel (1). Der Staubzucker wird mit einer Geschwindigkeit von etwa 20 Touren pro Minute gerührt.

Das Wasser wird zunächst kondensieren, wird aber bei Erhöhung der Temperatur des Staubzuckers durch Kondensationswärme auf 70 bis 75 Grad C wiederum verdampfen und im Kondensator (2) kondensieren. Man beobachtet die Granulatentwicklung am Vakuumtrockner. Bei genügender Granulatbildung wird das Ventil (4) geschlossen und die Vakuumpumpe auf volle Vakuumleistung gestellt, wodurch das in den Granulatkörnern verbliebene Wasser entfernt wird und ein trockenes Granulat entstanden ist.

**Beispiel 2:**

In einen Vakuum-Mischkessel werden 200 kg Lactose und 6 kg Polyvinylpyrrolidon mittlerer Kettenlänge eingebracht und vermischt. Das Produktgemisch wird im Vakuummischer bei einer Manteltemperatur von 70 Grad C auf 50 bis 55 Grad C erhitzt.

Im Lösungsmittelzuführgerät (6) befinden sich 15 l Alkohol. Wie unter Beispiel 1 wird diesmal die Wärmeaustauschertemperatur auf 120 Grad C gebracht, der Kompressorüberdruck wiederum auf 0,2 bis 0,5 bar, das Vakuum aber auf 600 mbar eingestellt. Man lässt unter diesen Bedingungen bei einer Rührintensität von 20 bis 30 U/Minute innerhalb von etwa 10 bis 15 Minuten 15 Liter Alkohol - über den Wärmeaustauscher (5) dampfförmig mit heisser Luft vermischt - durch das Lactose/PVP-Gemisch strömen. Da die Kondensation von Alkohol etwas anders erfolgt als von Wasser, gelingt dieses Einströmen besonders vorteilhaft durch den perforierten Lochrührer. Bei Einströmen im Boden des Vakuummischers durch die Säule muss die Zeit auf etwa 20 Minuten erhöht werden, da das Einströmen langsam erfolgen muss. Bei Erreichung der gewünschten Granulatgrösse wird wiederum bei (4) abgestellt und durch Anlegen von vollem Vakuum getrocknet.

**Beispiel 3** (Partielle Entwässerung von Lactose):

Es werden 200 kg kristallwasserhaltige Lactose auf 80 Grad C erhitzt und 20 Liter Isopropanol über das Lösungsmittelzuführgerät eingebracht.

Bei einer Temperatur des Wärmeaustauschers von 90 Grad C und einem Druck von etwa 300 mbar wird wiederum Heissluft über den Kompressor bei 0,2 bis 0,5 bar durch das System gesaugt. Durch Öffnung des Hahnes (9) lässt man Isopropanol in einem Zeitraum von etwa 15 bis 20 Minuten durch die Lactose streichen, wobei wiederum ein Teil des Isopropanol kondensieren wird. Durch die freiwerdende Verdampfungswärme erhöht sich die Produkttemperatur, so dass nachfolgend Isopropanol wiederum verdampft und in (2) kondensiert wird.

Nach Beenden des Vorganges trocknet man wieder durch Anlegen des Vakuums und erhält somit ein Produkt, das zu etwa 30 % aus wasserfreier Lactose besteht. Dieses Gemisch wasserfreier und wasserhaltiger Lactose ist direkt zu Tabletten verpressbar und entspricht den bekannten, direkt verpressbaren Gemischen zwischen alpha- und beta-Lactose.

**Beispiel 4** (Entwässerung von Ampicillin):

200 kg Ampicillin werden bei einer Manteltemperatur von 40 Grad C, einem vorgelegten Vakuum vom 800 mbar und einem Ueberdruck am Kompressor von 0,2 bis 0,5 mbar mit Heissluft von 130 Grad C am Wärmeaustauscher (5) durchströmt. Im Lösungsmittelzuführgerät (6) befinden sich 60 Liter Isopropanol, das mit einer Geschwindigkeit von 20 Liter pro Stunde durch den Wärmeaustauscher verdampft und durch das kristallwasserhaltige Ampecillin - nur teilweise kondensierend - hindurchsaugt wird. Nach Verdampfen des gesamten Isopropanols und Endtrocknung durch Anlegen von Vollvakuum ist der Kristallwassergehalt des Ampicillins von etwa 13,4 % auf 0,4 % gesunken.

**Beispiel 5** (Stärkegranulation):

Im Vakuumkessel werden 160 kg Lactose und 20 kg Stärke vorgelegt. Das Gemisch wird bei einer Manteltemperatur von 100 Grad C auf 80 Grad C erhitzt. Man bereitet aus 20 l Wasser und 20 kg Stärke eine Suspension und saugt diese mittels eines Dreiweghahnes beim Ventil (4) ein. Nach Mischen mit 30 U/Minute saugt man 10 Liter Wasser im Lösungsmittelzuführgerät (6) ein, wobei die Wärmeaustauschertemperatur 120 Grad C, der Kompressorüberdruck 0,2 bis 0,5 bar, das Vakuum im Kessel etwa 700 mbar und die Einströmdauer ca. 10 Minuten betragen.

Durch Anlegen des vollen Vakuums entsteht ein trockenes, überaus gleichförmiges Granulat von Stärke/Lactose.

Dieses Verfahren eignet sich nicht unbedingt für den perforierten Lochrührer, da bei der Entstehung des Stärkekleisters die Löcher im Rührer verklebt werden können. Dieses Verfahren wird vorteilhaft durch Einsaugen am Boden des Gefässes durchgeführt.

**Patentansprüche**

1. Verfahren zur Wärmebehandlung von in einer geschlossenen Trommel in Bewegung befindlichem Pulver oder Granulat, bei dem ein heisser Gasstrom durch das Behandlungsgut hindurch getrieben wird, dadurch gekennzeichnet, dass der Gasstrom vor seinem Eintritt in die Trommel mit einem dampfförmigen Behandlungsmittel beladen wird.

2. Verfahren nach Anspruch 1, dadurch gekennzeichnet, dass der Gasstrom durch zumindest ein Rohr durch das Behandlungsgut geleitet und anschliessend in feiner Verteilung an vielen Stellen - vorzugsweise nahe der Trommelwand - auf das Behandlungsgut trifft.

3. Verfahren nach Anspruch 1 oder 2, dadurch gekennzeichnet, dass in der Trommel - wenigstens während eines Teiles des Verfahrensablaufes - ein Unterdruck von etwa 0,1 bis etwa 0,4, vorzugsweise von 0,2 bis 0,3 bar, herrscht.

4. Verfahren nach einem der vorangehenden Ansprüche, dadurch gekennzeichnet, dass man ein aus wenigstens einem Behandlungsmittel und einem Trägergas bestehendes Gemisch von einer Temperatur, bei welcher das Behandlungsmittel in Dampfform vorliegt, durch das in Mischbewegung gehaltene Bett des Gutes, welches sich auf einer Temperatur unter der Siedetemperatur des Behandlungsmittels und bei annähernd Normaldruck befindet, hindurchsaugt und sodann nach der gewünschten Einwirkungsdauer das auf dem Gut kondensierte Behandlungsmittel durch Anwendung eines Unterdruckes, der bei oder unterhalb des Druckes liegt, der dem Siedepunkt des Behandlungsmittels bei der Temperatur des Gutes entspricht, aus dem Gut verdampft und aus dem Behandlungsraum absaugt.

5. Verfahren nach einem der vorangehenden Ansprüche, zurgegebenenfalls partiellen - Entwässerung kristallwasserhältigen Behandlungsgutes, dadurch gekennzeichnet, dass als Behandlungsmittel ein Alkohol, vorzugsweise Isopropanol, eingesetzt wird.

## Claims

1. Process for the heat-treatment of powder or granules in motion in a closed drum, in which a hot stream of gas is forced through the material being treated, characterized in that the gas stream, before entering the drum, is laden with a treatment agent in vapour form.

2. Process according to Claim 1, characterized in that the gas stream is passed through at least one pipe through the material being treated and then comes into contact, in finely divided form and at many points, preferably close to the drum wall, with the material being treated.

3. Process according to Claim 1 or 2, characterized in that reduced pressure of about 0.1 to about 0.4, preferably 0.2 to 0.3, bar is maintained in the drum, at least during part of the procedure.

4. Process according to any of the preceding Claims, characterized in that a mixture which consists of at least one treatment agent and a carrier gas and is at a temperature at which the treatment agent is present in vapour form is sucked through the bed of material, which bed is kept in motion with mixing and is at a temperature below the boiling point of the treatment agent and under approximately atmospheric pressure, and then, after the desired duration of action, the treatment agent condensed on the material is vaporized from the material by applying reduced pressure which is at or below the pressure corresponding to the boiling point of the treatment agent at the temperature of the material, and the said treatment agent is extracted from the treatment space by suction.

5. Process according to any of the preceding Claims, for partial or complete dehydration of material to be treated and containing water of crystallization, characterized in that an alcohol, preferably isopropanol, is used as the treatment agent.

## Revendications

1. Procédé destiné au traitement thermique d'un produit pulvérulent ou granulé, se trouvant en mouvement dans un tambour clos, dans lequel un courant gazeux chaud est amené à travers le produit en cours de traitement, caractérisé en ce que le courant gazeux est, avant son entrée dans le tambour, chargé d'un agent de traitement à l'état de vapeur.

2. Procédé selon la revendication 1, caractérisé en ce que le courant gazeaux traverse le produit à traiter par au moins un tuyau, puis est dirigé sous forme finement divisée, en de multiples endroits, situés de préférence à proximité de la paroi du tambour, sur le produit en cours de traitement.

3. Procédé selon la revendication 1 ou 2, caractérisé en ce que dans le tambour, au moins pendant une partie de l'opération de traitement, il règne une dépression d'environ 0,1 à environ 0,4 et de préférence à 0,2 à 0,3 bar.

4. Procédé selon l'une quelconque des revendications précédentes, caractérisé en ce que l'on aspire à travers le lit du produit à traiter, porté à une température inférieure à la température d'ébullition de l'agent de traitement, à une pression voisine de la pression normale et maintenu en mouvement tourbillonnant, un mélange formé d'au moins un agent de traitement et d'au moins un gaz porteur à une température, à laquelle l'agent de traitement est à l'état de vapeur, et, après la durée du traitement prévu, on élimine l'agent de traitement condensé sur le produit traité par application d'une dépression égale ou inférieure à la pression, qui correspond au point d'ébullition de l'agent de traitement à la température du produit, et on le soutire de l'enceinte de traitement par aspiration.

5. Procédé selon l'une quelconque des revendications précédentes, pour la déshydratation éventuellement partielle du produit en cours de traitement contenant de l'eau de cristallisation, caractérisé en ce qu'en tant qu'agent de traitement on met en oeuvre un alcool, de préférence de l'isopropanol.

Fig. 1

Fig. 2